# EUROPEAN PATENT APPLICATION

(11) **EP 4 772 147 A1**
(43) Date of publication of application: **08.07.2026**
(21) Application number: 25227558.1
(22) Date of filing: 30.12.2025
(51) Int. Cl.: A61F 2/44, A61B 34/10, A61F 2/30

(54) **CUSTOM, PATIENT-SPECIFIC IMPLANTS AND METHODS OF MAKING THEREOF**

(30) Priority: 03.01.2025 US 202563741585 P; 05.02.2025 US 202519045953
(71) Applicant: Globus Medical, Inc., Audubon, PA 19403 (US)
(72) Inventor: YACOUB, George, LANSDALE, 19446 (US); JOHNSON, Norbert, NORTH ANDOVER, 01845 (US); JOSHI, Sanjay, ANDOVER, 01810 (US); RAPP, Cassie, PHOENIXVILLE, 19460 (US); GONEAU, Caleb, PHILADELPHIA, 19104 (US); SEIFERT, Jody L., BIRDSBORO, 19508 (US); LORMAN, Bess, HARLEYSVILLE, 19438 (US); CRAWFORD, Neil, CHANDLER, 85224 (US)
(74) Representative: Morabito, Sara

(57) **Abstract**

Custom patient-specific interbody implants, systems, and methods of generating custom implants. The implants may include upper and lower surfaces with an irregular surface topography, which are custom contoured to match the adjacent vertebral endplates of a specific patient. The method may include uploading images of the spine for the patient and processing the images into a virtual three-dimensional model of the spine including topography of individual vertebral bodies. The user may automatically and/or manually manipulate the virtual three-dimensional model to pre-operatively simulate a desired correction to the spine. The virtual implant model may be generated based on the desired correction and the topography of adjacent vertebral bodies.

## Description

### CROSS REFERENCE TO RELATED APPLICATIONS

This application claims priority to U.S. Provisional Application No. 63/741,585, filed on January 3, 2025, which is incorporated by reference herein in its entirety.

### FIELD

The present disclosure relates to surgical devices, systems, and methods, and more particularly, customized patient-specific implants that are tailored to fit a given patient, and methods for making the same.

### BACKGROUND

The current spine industry offers a variety of implants that can be used in the treatment of various spinal pathologies. A common procedure for handling pain associated with intervertebral discs that have become degenerated due to various factors such as trauma or aging is the use of intervertebral fusion devices for fusing one or more adjacent vertebral bodies. Generally, to fuse the adjacent vertebral bodies, the intervertebral disc is first partially or fully removed. An intervertebral fusion device is then typically inserted between neighboring vertebrae to maintain normal disc spacing and restore spinal stability, thereby facilitating an intervertebral fusion.

There are multiple parameters surgeons are looking to improve during a spinal fusion procedure, such as restoration of disc height for indirect decompression of the neural foramen, coronal and sagittal balance to help distribute the body's weight evenly to reduce stress on various parts of the spine and maintain overall balance. The potential degree of available correction is dependent on the available interbody spacers and their geometries as well as the individual patient's anatomy. Many of these spacers are offered in various pre-determined shapes and sizes, including medial-lateral width, anterior-posterior depth, cephalad-caudal height, and lordotic and coronal tapers. These available spacers also have standard endplate surfaces.

Many surgeons may create a pre-operative plan based on patient images to help determine the correction of the spine they wish to achieve and potential spacer options that could help them achieve that goal. Corrections may be limited, however, by the spacers available, thereby limiting potential surgical outcomes.

### SUMMARY

Described herein are implants, systems, and methods which provide for tailored and customized patient-specific implants. The custom interbody implants, methods, and systems may be optimized for determining and creating custom interbody implants for a given patient. As explained above, surgeons are currently limited to pre-determined sized and shaped implants. There currently does not exist an offering that allows the surgeon to design a patient-specific implant based on the pre-operative plan and vertebral body topography to create an implant with specific dimensions required to achieve the custom, pre-operative plan. Allowing a surgeon to design an implant specific to the patient is especially critical for complex procedures where the pre-determined shape and size implants currently available may not fit well into the disc space and/or are unable to restore the spine to achieve optimal global alignment. This is especially relevant for patients who have atypical vertebral body morphology, such as highly-scalloped endplates or wedge-shaped or rotated vertebral bodies.

Accordingly, the design and manufacturing of surgeon-designed patient-specific implants may include a process for modeling the custom implants, for example, based on patient imaging and the pre-operative plan. The first step in the process may include uploading images of a patient's spine into a secure portal. Within that portal, the surgeon can select the operative spinal levels and the chosen approach angle. The surgeon can also input specific dimensional parameters for the proposed implant in order to achieve their desired surgical plan. For example, such parameters can include, but are not limited to, static v. expandable spacer, anterior-posterior depth, medial-lateral width, anterior height, posterior height, lordotic angle, and coronal angle. Additionally, if designing an expandable spacer, the surgeon can select a desired starting height based on any known anatomy or access limitations. Further, many surgeons have set parameters for each level of the spine and approach. The methods, processes, and software described herein can allow each surgeon to maintain a profile containing their preferred parameters and allow them to auto-select said parameters for each case, minimizing the time required to design the implant.

Because the implants are designed for a specific anatomical location in a specific patient, three-dimensional (3D) models may be developed of a virtual implant that can be used to both instruct the manufacturing of the implant and show the surgeon their pre-operative plan within a software module. The software module may allow surgeons to upload imaging, such as computed tomography (CT) scans and standing x-rays, segment the CT scan into a usable 3D model, and allow the surgeon to manipulate the location of spinal elements to a desired correction. A virtual implant model may be generated based on the correction and patient's vertebral body topography. The virtual implant can then also be used to plan a surgical procedure including approach trajectory and necessary bony work to achieve the desired correction.

In another embodiment, once the scans are uploaded and desired implant parameters are input, a modeling team can implement those design parameters in modeling software or computer aided-design (CAD) to output a virtual spacer designed by the surgeon with patient-matched inferior and superior topography. This may involve segmenting the patient images to create the pre-operative model of the spine. The modeling team may then create the virtual spacer, which is tailored for the given patient. These virtual implants can then be placed in the 3D model of the spine to simulate the desired correction of the spine.

Once the models are generated, the pre-operative 3D rendering of the spine, designed implants, corrected 3D rendering of the spine, implant parameters, and pre-planned segmental and global spinal parameters can then be communicated back to the surgeon. Segmental and global spinal parameters can include, but are not limited to, lumbar lordosis, segmental lordosis, coronal angle, pelvic incidence, anterior disc height, posterior disc height, and posterior foraminal height. The surgeon can communicate any adjustments for the software or modeling team to implement and/or can provide final approval.

In some embodiments, when the surgeon is uploading the scans, he or she can design the implant by inputting desired surgical outcomes including lumbar lordosis, segmental lordosis, coronal angle, pelvic incidence, anterior disc height, posterior disc height, and posterior foraminal height with the option to have the implant modeled to achieve those parameters. The software disclosed herein can also include measurement tools to allow the surgeon to measure the pre-operative parameters in order to guide their decision in post-operative spinal parameter goals.

In some embodiments, when the surgeon uploads the scans, the images may be segmented within the portal into a 3D rendering of the pre-operative spine. The surgeon can then manipulate the vertebral bodies in the x, y, and z directions to help guide decisions on what the post-operative spinal parameter goals are and/or the implant parameters necessary to achieve said goals.

In one embodiment, a method of creating a custom patient-specific interbody implant for a patient may include: (a) uploading images of a spine for the patient into a processor configured to execute software instructions stored on memory; (b) processing the images into a virtual three-dimensional model of the spine including topography of individual vertebral bodies; (c) displaying the virtual three-dimensional model of the spine on an interface connected to the processor; (d) manipulating the virtual three-dimensional model on the interface to pre-operatively simulate a desired correction to the spine; (e) generating a virtual implant model based on the desired correction to the spine and the topography of adjacent vertebral bodies; and (f) using the virtual implant model to plan and/or show a surgical procedure to achieve the desired correction of the spine tailored for the patient. The imaging may include lying CT scans and standing X-rays of the patient. Based on the imaging, the virtual three-dimensional model of the spine may be automatically modified to represent a standing posture instead of a lying posture of the patient. When manipulating the virtual three-dimensional model on the interface, any potential collisions between adjacent bones may be indicated on the virtual three-dimensional model of the spine. The virtual three-dimensional model may include a series of meshes in three mutually orthogonal multi-planar reconstruction views, and the virtual implant model may include a mesh reshaped by the virtual three-dimensional model of the spine. The virtual implant model may be generated by overlapping a portion of the mesh of the virtual implant model with a portion of the series of meshes of the virtual three-dimensional model such that any intersecting areas are subtracted from the mesh of the virtual implant model to mimic the topography of adjacent vertebral bodies. The method may also include: (g) manufacturing a custom interbody implant for the patient based on the virtual implant model; and (h) installing the custom interbody implant into the patient based on the virtual implant model and the desired correction.

In one embodiment, a custom patient-specific interbody implant includes a body having an upper surface and a lower surface configured to contact adjacent vertebral bodies. The upper and lower surfaces each have an irregular surface topography, which are custom contoured to fit between adjacent vertebral endplates of a specific patient. The irregular surface topography is predetermined from a virtual implant model which is generated by overlapping a portion of the virtual implant model with a portion of a virtual three-dimensional model of a spine of the patient, simulated pre-operatively with a desired correction to the spine, such that any intersecting areas are subtracted from the virtual implant model to mimic the topography of the adjacent vertebral endplates of the patient. The virtual three-dimensional model of the spine of the patient is simulated based on segmentation of pre-operative two-dimensional and three-dimensional imaging of the patient, thereby converting the virtual three-dimensional model of the spine to represent a standing posture instead of a lying posture of the patient. The upper and lower surfaces may be constructed from a mapped topography of the adjacent vertebral endplates for the specific patient and filled in with a trabecular lattice. The implant may be a static implant or an expandable implant. The expandable implant may include an upper endplate defining the customized upper surface and a lower endplate defining the customized lower surface, and a central actuator with ramps configured to expand the upper and lower endplates. The upper and lower endplates may be each represented in the virtual implant model as two separate meshes including a modifiable mesh portion that is subtracted to form the irregular surface topography and an unmodifiable mesh portion that is retained for structural integrity of the upper and lower endplates. Alternatively, the implant may include a solid shell surrounding an inner porous center.

In one embodiment, a method of generating a custom implant includes: (a) uploading two-dimensional and three-dimensional images of a portion of a patient's spine; (b) processing the images on a processor configured to execute software instructions stored on memory; (c) registering the images; (d) creating a patient-specific kinematic model that estimates joint forces of each motion segment of the patient's spine; (e) automatically suggesting spinal modifications; (f) manually modifying the automatically suggested spinal modifications and/or accepting the automatically suggested spinal modifications; and (g) generating a custom implant based on the automatically suggested spinal modifications including any manual modifications. The step of processing the images may include detecting patient anatomy and/or regions of interest and automatically measuring global and local spine parameters. The step of registering the images may include registering two anatomical plane images to one origin to establish an initial three-dimensional coordinate system and then segmented levels are registered to the initial three-dimensional coordinate system. The step of automatically suggesting spinal modifications may include software instructions, which provide a corrected spine model based on a machine-learning model. The patient-specific kinematic model may be determined from the images and inputs concerning the patient including demographic data, anthropometric data, and physiological and biological data.

Also disclosed herein are custom anterior lumbar interbody fusion (ALIF) spacers and methods of inserting custom ALIF spacers. ALIF spacers often come with the option of integrated fixation through the spacer itself. Integrated fixation offers surgeons the benefit of stabilizing the spine via an anterior approach, potentially eliminating the need for additional posterior surgery. This benefits the patient by reducing anesthesia time, blood loss, and muscle disruption. While patient specific implants may help restore disc height and spinal alignment, patients with complex anatomical deformities may present a challenge for both ALIF interbody implant insertion and fixation insertion due to limited access angles or vascular tissue, as the aorta and vena cava are located anterior to the spine and need to be mobilized for an ALIF procedure.

### BRIEF DESCRIPTION OF THE DRAWINGS

FIG. 1 is a flowchart representative of a method for pre-operatively determining a three-dimensional model of a patient's spine and generating a virtual implant model based on the desired correction and patient's unique vertebral topography, according to some embodiments;
FIG. 2 is a flowchart representative of a method for creating and installing custom interbody implant(s) that are modeled for a specific patient, according to some embodiments;
FIG. 3A shows an initial shape of an extracted spine model, for example, based on patient imaging for a specific patient, according to some embodiments;
FIG. 3B shows a target shape of the spine shape model for a desired spinal correction for the patient, according to some embodiments;
FIG. 4A shows a hybrid polyrigid deformation and mesh representation before manipulation of the vertebrae, for example, based on imaging of a specific patient, according to some embodiments;
FIG. 4B shows the hybrid polyrigid deformation and mesh representation after virtual manipulation of the vertebrae to achieve the desired correction, according to some embodiments;
FIG. 5 shows an example of user input controls for entering deformations at selected level(s) of the bones, according to some embodiments;
FIG. 6A shows user input controls for manually entering desired manipulations to the vertebrae on the virtual spine model, according to some embodiments;
FIG. 6B shows user input controls after desired manipulations to the vertebrae on the virtual spine model, according to some embodiments;
FIG. 7A shows a representation of a spinal segment of the virtual spine model, according to some embodiments;
FIG. 7B shows a representation of the spinal segment made semi-transparent to allow for visualization of potential collision areas and with indicators of potential bony collision(s), according to some embodiments;
FIG. 8 shows a flowchart representing the process for uploading patient imaging and changing spinal alignment of the virtual spine model, according to some embodiments;
FIG. 9A shows a device mesh blank that is sized slightly taller than the disc space of the virtual spine model to determine the virtual implant model shape, according to some embodiments;
FIG. 9B shows the surrounding bone meshes subtracted from the device mesh blank, creating a virtual interbody implant with contoured surfaces contacting the vertebral endplates at the desired height and angle, according to some embodiments;
FIG. 9C shows a resulting patient-specific interbody implant with custom contoured upper and lower surfaces configured to mimic the vertebral endplates of the adjacent vertebrae of the patient, according to some embodiments;
FIG. 10A shows a method for generating a custom expandable implant, wherein the top and bottom expanding pieces are each represented as two separate meshes including modifiable and unmodifiable meshes, according to some embodiments;
FIG. 10B shows the top and bottom expanding pieces positioned where the unmodifiable meshes are just on the brink of intersecting with the bone meshes;
FIG. 10C shows that when the upper and lower meshes are positioned in the target location, the bone meshes are subtracted from the modifiable meshes, leaving contoured upper and lower endplates for the expandable implant, according to some embodiments;
FIG. 11 shows a process for automatically creating a porous device with an outer reinforcing shell, according to some embodiments;
FIG. 12 is a flowchart representative of an implant shaping stage for creating the virtual interbody implant, according to some embodiments;
FIGS. 13A and 13B show a virtual spine model for a specific patient in its existing state and with a virtual correction, respectively, according to some embodiments;
FIG. 14 shows mapping topography of a vertebral endplate for a specific patient, according to some embodiments;
FIGS. 15A-15C show a process of endplate creation for an expandable interbody implant including applying bone-matched morphology to an endplate blank, for example, with a lattice structure, according to some embodiments;
FIG. 16 shows an assembled expandable interbody implant with patient-matched custom upper and lower endplates, according to some embodiments;
FIG. 17 shows a scaling calibration method between a CT scan of a patient with known scaling and a radiograph of the patient with unknown scaling to obtain a calibrated radiograph; and
FIG. 18 shows a scaling calibration method between radiographs of a patient with known and unknown scaling to obtain a calibrated radiograph.

### DETAILED DESCRIPTION

Treating spine pathologies often cannot be boiled down to a simple set of rules that works for all patients. Ideal shapes of the spine are well documented, but how to achieve those ideal shapes are not as well defined. Custom implants can help achieve those alignments by distributing coronal and sagittal correction with a single custom made interbody that conforms to the patient's anatomy, giving them the best opportunity to heal.

The benefits of the custom implants and methods described herein include enabling surgeons to plan surgery using a custom implant created from patient's anatomical images, enabling integrated delivery of CAD model of custom implant, and enabling intra-operative evaluation of progress of surgery compared to the plan.

Allowing surgeons to design each implant for a specific patient's anatomy, pathology, level, and approach, in combination with patient-matched topography on the superior and inferior surfaces of the implants, better allow a surgeon to achieve his or her pre-operative planning goals and help achieve better global alignment of the spine. Restoring spinal alignment reduces post-surgical complications including proximal junctional kyphosis, proximal junctional failure, and implant-related complications. These complications can cause the need for additional surgery, putting the patient at increased risk for surgical complications including blood loss and/or infection. The patient-matched topography on the superior and inferior implant surfaces maximizes contact area between the spacer and the patient's bone, which increases implant stability and reduces the risk of subsidence.

Additionally, involving the surgeon earlier on in the implant design process familiarizes them with the implant(s) and aids them in the pre-operative planning process. Knowing precisely what implant they are receiving helps the surgeon plan for and avoid any complications that may arise due to implant geometry. Considering patient anatomy, creating a solution, and then seeing exactly how that solution is generated gives the surgeon a much more informed approach to their patient and the issue they are solving.

The software presented herein provides a clear and easy interface for planning these patient-specific interbodies and gives the user control of the process. The software also shows standing spinal posture instead of posture lying supine in the CT scanner and provides real-time feedback to the user on how the spine will be configured when they insert implants shaped to their plan.

Having the option of expandable implants may also reduce the likelihood of post-surgical complications. Expandable implants reduce the need for trialing for implant height, reducing instrument passes to the surgical site, reducing surgical time and therefore anesthesia time, as well as reducing risk of infection. They are also inserted at a shorter height to help reduce endplate trauma, which may also reduce the risk of subsidence.

Generic implant parameters (lordosis angle/width/length) often do not allow a surgeon to correct multiple planes with one single implant. For those interbodies that do allow for multiple plane manipulation, the endplates may not adequately conform to the patient. Additionally, those interbodies may not enable a reliable target correction as they may not match directly with goal correction. This method describes how to automatically generate a custom interbody to fit the patient's anatomy and achieve the surgeon's goal. The methods described herein address the above challenges by autogenerating custom interbodies to fit the needs of the preferred surgical workflow and patient anatomy.

Surgeons may develop a pre-operative plan using patient imaging to help determine the desired spinal correction, surgical approach, implant type, size, etc. In some cases, pre-operative planning can be performed using a surgical robotic and/or navigation system equipped with a processor designed to run software instructions stored on its memory. This system typically includes a user interface, such as a touch screen, allowing for interactive planning and simulation of surgical procedures. Examples of surgical robotic and/or navigation systems can be found, for example, in U.S. Patent Publication No. 2019/0021795 and U.S. Patent Publication No. 2017/0239007, which are incorporated by reference herein in their entireties for all purposes. It will be appreciated that any suitable operating system and user interface may be selected for this purpose.

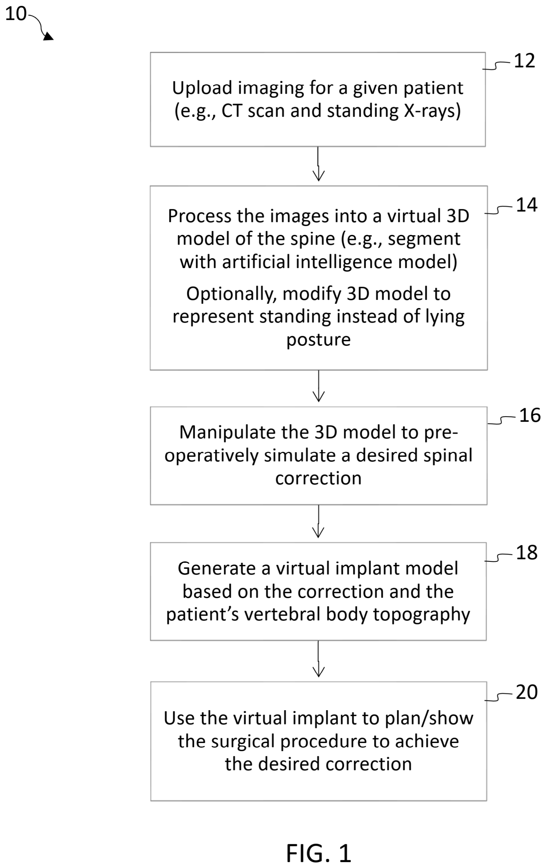

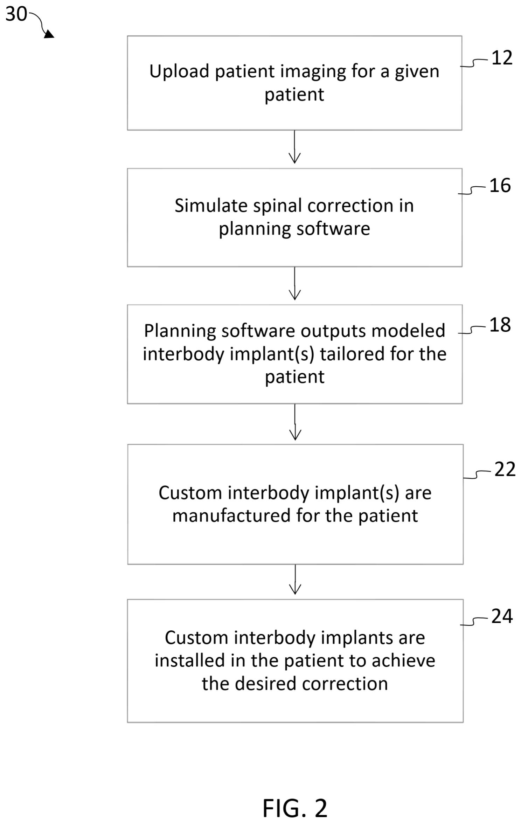

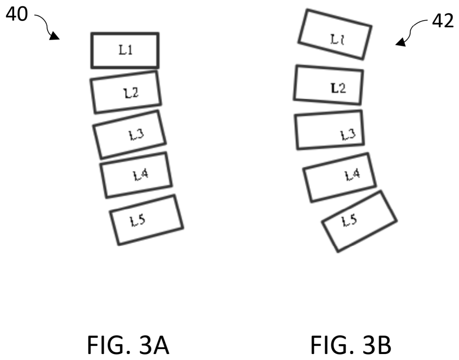

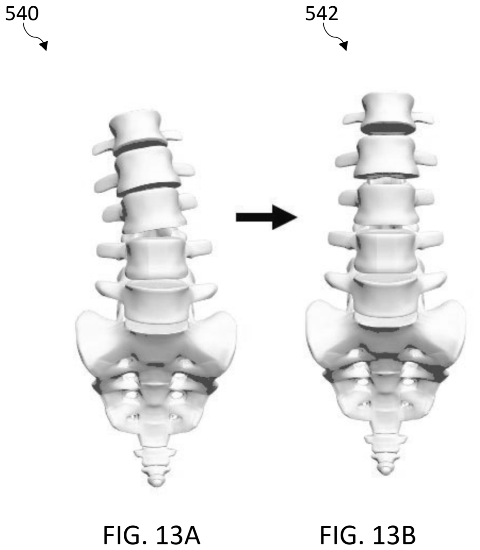

Turning now to the drawings, FIGS. 1 and 2 illustrate flowcharts 10, 30 for creating custom interbody implant(s) for a specific patient, according to some embodiments. FIG. 1 is a flowchart 10 representative of a method for pre-operatively determining a three-dimensional model of a patient's spine and generating a virtual implant model based on the desired correction and patient's unique vertebral topography. FIG. 2 is a flowchart 30 representative of a method for creating and installing custom interbody implant(s) that are modeled for a specific patient. FIG. 3A shows an initial shape of an extracted spine model 40, for example, based on patient imaging for that specific patient, and FIG. 3B shows a target shape of the spine shape model 42 for a desired spinal correction. Similarly, FIG. 13A shows an initial spine model 540 for the patient, and FIG. 13B shows the desired spinal correction 542 for coronal and sagittal balance. It will be appreciated that any suitable spine model may be used or generated for this purpose.

Referring to FIGS. 1 and 2, at step 12, imaging for a given patient may be uploaded into the system (e.g., CT scans and/or X-rays). Next, at step 14, the images are processed into a virtual 3D model of the patient's spine or a portion thereof. At step 16, the 3D model is manipulated, for example, automatically by the system/software and/or by the user to pre-operatively simulate a desired spinal correction for the patient. In some embodiments, the portal software provides the corrected spine model with the desired spinal correction, for example, based on artificial intelligence (AI) and/or a machine-learning (ML) model. The AI algorithms may include supervised learning, unsupervised learning, semi-supervised, reinforcement learning, or other suitable algorithms. The system may process and analyze large volumes of data, extract insights, predict trends, and learn from new data inputs over time to optimize outcomes. It will be appreciated that any suitable AI algorithms or machine learning models may be used based on the most appropriate methodologies. The surgeon can then manually manipulate the corrected spine model to achieve the desired correction, if warranted, to accommodate surgeon preference. The software may inform the surgeon if their manual correction requires additional surgical intervention. The software provides a clear and easy interface for planning patient-specific interbody implant(s) and gives the user control of the process.

The system generates a virtual implant model at step 18 based on the desired spinal correction and the patient's vertebral topography. In other words, the software generates the interbody implant(s) needed to complete the surgical plan. At step 20, the virtual implant model from step 18 may be used to plan and/or show the surgical procedure in order to achieve the desired spinal correction for the patient. The Software may generate a surgical procedure starting from the virtual implant. The surgical procedure being output and/or displayed.

The virtual implant can then be placed in the 3D model of the spine to simulate the desired correction of the spine. The results of the simulation may be displayed or output from the software.

Referring specifically to FIG. 2, after the interbody implants are modeled at step 18, the custom interbody implant(s) may be manufactured at step 22 for the patient (e.g., via 3D printing). The custom interbody implant(s) are then installed at step 24 in the patient to achieve the desired correction as planned. The implant(s) may be implanted freehand by the surgeon or with the assistance of robotic and/or navigational systems.

These various processing steps are described in more detail below.

Image Upload: In the first step 12, images of a patient's spine are uploaded into a secure portal. The images may include 2D and/or 3D scans of the patient's spine. In some embodiments, a user uploads both 2D and 3D scans of a patient in a computer program. The 3D scan may be a computed tomography (CT) and/or a magnetic resonance imaging (MRI) scan, for example. The 2D scan may be in any image format (JPEG, PNG, etc.) as well as DICOM (EOS-Xray or Xray). Once uploaded, the user can select which region of the spine the software should continue to recognize for image processing. The 2D images may show the spine position under a loaded condition as the patient is standing when the image(s) are taken, and standing puts weight on the spine and can compress the spinal discs. The 3D images may show the patient in a lying position with the spine in a supine position with neutral spine alignment.

Image Processing: In the second step 14, the uploaded images are processed. The 2D images may be processed using a trained neural network (e.g., an AI model trained using hundreds of labeled images). In some embodiments, the system/software can automatically detect the image plane to distinguish between an anteroposterior (AP) and a lateral image. The system can automatically recognize anatomy presented and/or the bony anatomy in sections of the image including, but not limited to, vertebrae, vertebral bodies, sacrum, ilium, clavicle, and femoral heads as well as their anatomical positions. Moving both from the sacrum up and the clavicle down, the system can then label the vertebrae to ensure proper classification (lumbar L4, L5, sacrum S1, thoracolumbar T12, etc.). Using a separate neural network or the same neural network, the system may detect specific regions within the anatomy of interest. Regions or anatomical landmarks of interest may include, but are not limited to, femoral head centers and/or four corners of a vertebral body in either plane.

The computer may then automatically measure global spine parameters and local spine parameters. Global spine parameters may include, for example, Cobb angle, lumbar lordosis (LL), thoracic kyphosis (TK) pelvic incidence-lumbar lordosis (PI-LL) mismatch, T1 pelvic angle (TPA), and sagittal vertical axis (SVA). Local spine parameters are specific to each operative level and may include, for example, anterior and posterior disc height, spondylolisthesis slip, vertebral body endplate width (mediolateral) and depth (anterior/posterior) for both the inferior and superior vertebral body endplates. Because magnification of untracked and unregistered X-rays is typically not known, parameters related to distances may not be expressed in millimeters, but may instead be expressed as proportions to other parts of the adjacent anatomy. For example, level-by-level spondylolisthesis (anterior shear of one vertebra over another) may be expressed as percentage of the vertebral body depth that protrudes anterior to the adjacent caudal vertebra.

The shape of the spine may be defined via position (e.g., 3D location and 3D rotation angles). The shape of spine can be visualized as a 3D CAD model or other suitable model. The computer can then automatically measure global spine parameters such as Cobb angle, lumbar lordosis, thoracic kyphosis, and local spine measurements such as anterior/posterior disc height. 3D images can provide greater detail at a local level compared to 2D images, but they provide limited insight into the final standing position (global position) of the patient.

The 3D images are also processed by the system. In some embodiments, the software recognizes bony anatomy including vertebrae, the sacrum, the ilium, and the like. The software then segments the bony anatomy into individual identified vertebrae creating an initial 3D model of a virtual spine with each vertebra modeled as a separate rigid body. In one embodiment, segmentation is performed manually, by scrolling through closely-spaced slices of the 3D volume and selecting pixels on each slice that belong to different vertebrae. Software for performing such manual segmentation may also be utilized (e.g., Mimics by Materialise or ScanIP by Synopsis). When using such software, pixels belonging to different vertebrae may be painted different colors on each slice. Then when all slices are painted, the volume is stacked together and continuous sections of the same color are joined, creating segmented 3D volumes. In another embodiment, segmentation can be performed using an artificial intelligence model such as a neural networks model that has been trained by providing CT scan volumes of spines that have been manually segmented. After segmentation, the virtual spine can be measured through software features and global and local spine parameters of the spine can be collected and displayed for the user if desired.

In some embodiments, the 2D image data set is further used to modify the 3D model of the spine to represent a standing instead of a lying posture. Although CT scans are typically taken when the patient is lying supine in the scanner, standing x-rays may also be taken contemporaneously for the patient. The segmental angles and linear offsets read automatically from X-rays as described above, or measured manually by marking and measuring X-rays, can be compared to the same angles and offsets in the 3D model and the differences necessary to correct the 3D model measured. These segmental corrections can be entered automatically or manually by the user or by software to configure the 3D model to represent the standing instead of lying posture. Such manipulation can occur before the user begins modifying the positions and angles of the vertebrae in 3D, described below. The software may show standing spinal posture instead of posture lying supine in the CT scanner and provide real-time feedback to the user on how the spine may be configured when they insert implants shaped to their plan.

Image Registration: In some embodiments, the uploaded images are then registered (i.e., image registration). For example, a computer may first register two anatomical plane images (e.g., AP and lateral planes) to one origin to establish an initial 3D coordinate system (e.g., establishing 0, 0, 0). Segmented levels from the uploaded 3D image are then registered to the coordinate system of the two anatomical plane images, morphing the 3D segments into the position of the individual vertebrae in the standing (loaded) position.

Informing Kinematic Model (Establishing ***k*):** The patient-specific kinematic model may be determined from the images and inputs concerning the patient including demographic data (e.g., age and gender), anthropometric data (e.g., height and weight), and physiological and biological data (e.g., bone quality). Given a patient's parameters (e.g., age, gender, height, weight, bone quality, etc.) and images, a patient-specific kinematic model may be created. The function of the kinematic model is to estimate joint forces of each motion segment. The kinematic model may be used to aid in the design of interventions by estimating the forces required to achieve the desired correction (see below).

Goal Setting (Establishing **ΔX**)**:** The computer may automatically suggest modifications of the spine to bring segmental parameters (local) and spinopelvic parameters (global) into global balance based on parameters, for example, described in literature and the expected segmental joint response (anatomical range of motion). The user then analyzes the suggestions and may then accept or modify it to their desired correction goal. Modifications may include additional or less sagittal, coronal, or axial correction both segmentally and/or globally. Additional refinements may include disc height (Anterior/Lateral) and (Left/Right).

Intervention Design (Minimizing **F** with **ΔX**): With the finalized correction goal(s) (auto generated or manual) resulting in target spine shape 42 (e.g., FIG. 3B), the specific interventions (such as interbody placement) needed to achieve those correction goals can be planned. Considering the final desired location of each vertebral body **(ΔX)** and updating the patient-specific kinematic model **(*k*),** the joint forces **(F)** required to achieve the surgical plan may be estimated. **(F = *k-* ΔX**)

To minimize the amount of force required to achieve the correction, interventions may be selected to minimize ***k*** while maintaining some level of stability (e.g., not simply suggesting a vertebral column resection). Interventions may include bone removal, disc removal, or soft tissue resection. In any area where disc is removed, an interbody may need to replace the disc to restore height and angle, as per the surgical plan.

Interbody placement may include the trajectory of surgical approach (Anterior, Lateral, or Posterior (TLIF, PLIF, unilateral, bilateral, etc.)) as well as final desired position of the spacer within the segment (e.g., more anterior, less anterior, at some oblique angle, etc.). Interbody placement may also be auto suggested due to anatomical site (for example, ALIF may be auto selected for the L5-S1 segment due to the interference of the iliac crest in a lateral trajectory, making lateral a poor surgical approach and the need for a hyper lordotic cage to achieve the surgical plan, making the posterior approach a poor surgical approach).

In some embodiments, the global spine parameters from a virtual 3D model can be presented to the user to aid in pre-operative planning. The user is then able to manipulate the posture of the spine graphically using a computerized interface via the flexion/extension angle, lateral bending angle, axial rotation angle, lateral shear, anteroposterior shear, and rostro-caudal compression/distraction. In some embodiments, the representation of the spine is a 3D series of meshes that can be colorized and shaded as desired to easily see perspective and to easily tell which vertebra is which. Such a representation resembles a 3D cartoon or CAD model of the spine. In some embodiments, the spine scan is displayed to the user in much the same way as the original scan was displayed, but using a polyrigid deformation algorithm, different sections of the scan volume can be moved relative to each other. In the polyrigid deformation algorithm, boundaries are formed around each vertebra within the scan using the segmentation data and then any portion of the scan that is within such a boundary is treated as a rigid body, meaning that it moves without deforming. Concurrently, any portions of the scan that are outside the boundaries set by segmentation are deformed as needed (stretched, compressed, twisted) to fill the intervening space.

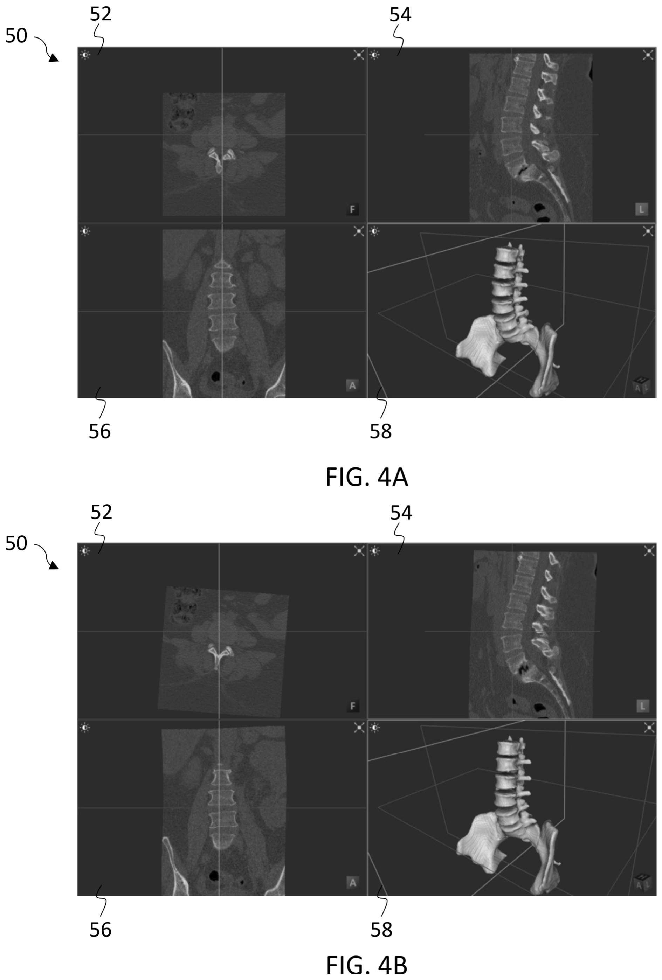

As best seen in FIGS. 4A-4B, a user display 50 may show a 3D view of the spine as a series of meshes and three mutually orthogonal MPR (multi-planar reconstruction) views represented using polyrigid deformation. FIGS. 4A and 4B show hybrid polyrigid deformation and mesh representation for showing realignment of vertebrae that the user has input through software. FIG. 4A shows one example of the initial virtual spine model before manipulating vertebrae. The system and/or the user may manipulate the 3D model to pre-operatively simulate a desired correction to the spine in step 16. FIG. 4B shows one example of the virtual spine model after manipulating vertebrae to obtain the desired correction. The images and manipulations would be unique for each patient. The three multi-planar reconstruction (MPR) views (top left, top right, bottom left of each of FIG. 4A and FIG. 4B) may show deformations as polyrigid deformation. In FIGS. 4A-4B, the top left view may include an axial view 52 of the virtual spine model, the top right view may include a sagittal view 54 of the virtual spine model, the bottom left view may include a coronal view 56 of the virtual spine model, and the bottom right view may include a 3D representation 58 of the virtual spine model with the position of the axial, sagittal, and coronal planes identified in the other views. The 3D view 58 (bottom right of each of FIG. 4A and FIG. 4B) shows spine realignment as movement of vertebra meshes. In either the 3D mesh embodiment or in the poly-rigid deformation embodiment, software controls can be used through which the user enters translation and rotation of the vertebrae in any direction and watches the deformations occur in real time. It will be appreciated that any suitable images or views may be displayed in any of the panels or areas on the user display 50.

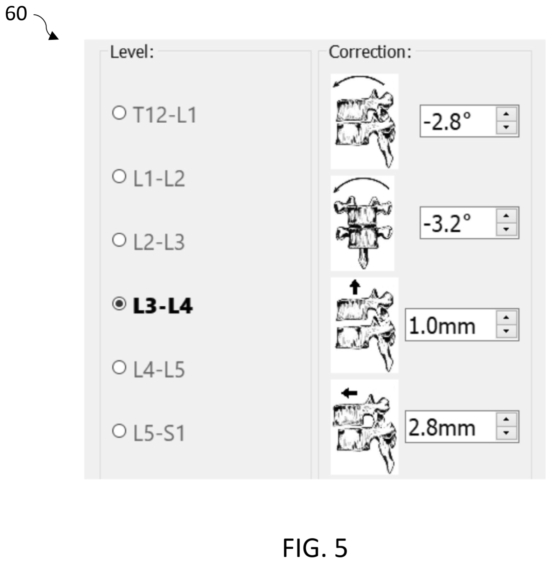

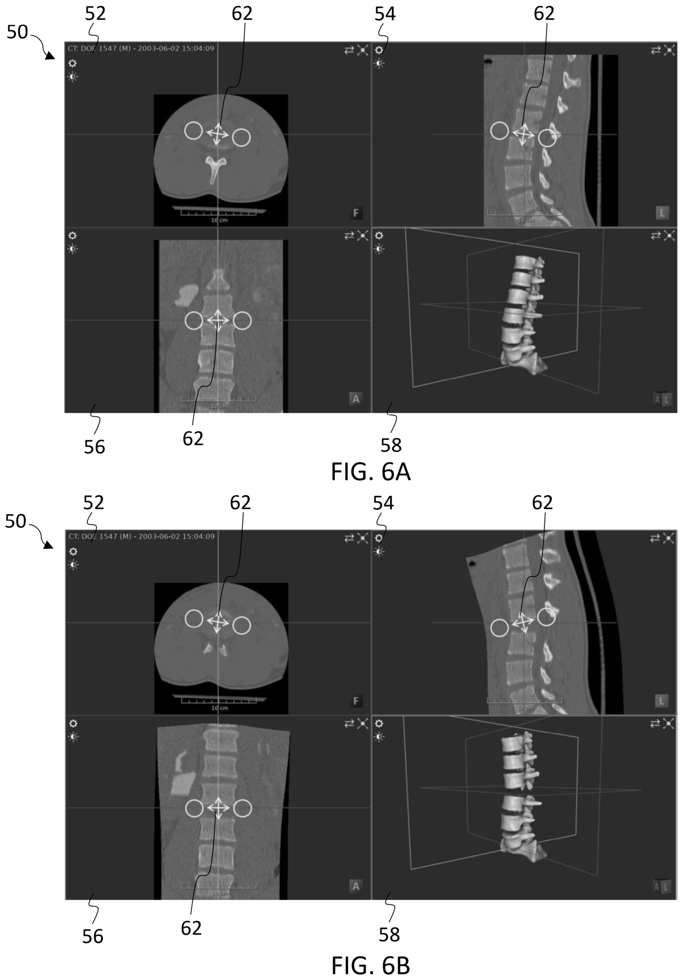

As angles and displacements of a vertebra occur across a selected level (L3-L4 in the example shown in FIG. 4A-4B), all levels rostral to that level remain unchanged in their position and orientation relative to the upper vertebra (L3) and all levels caudal to the selected level remain unchanged in their position and orientation relative to the lower vertebra (L4). In some embodiments, software controls 60 may be provided to the side, with scroll bars and text entry boxes allowing the user to set values for segmental angles and linear offsets (e.g., as shown in FIG. 5). In some embodiments, user input interaction handles 62 are overlaid directly over the representation of the spine, allowing the user to more directly move the handles through touchscreen or mouse interaction (e.g., as shown in FIG. 6A-6B).

More specifically, FIG. 5 shows an example of user input controls 60 for entering deformations at a selected level (L3-L4 is selected in the example shown) of the bones. In some embodiments, a user enters changes in angles and translations by typing values or toggling up/down arrows. Shown are flexion/extension, lateral bending, rostrocaudal distraction/compression, and anteroposterior shear controls. In some embodiments, controls can also exist for axial rotation and lateral shear. When changing values in such controls, responses could be either in the global coordinate system of the whole spine or in the local coordinate system of the selected level (L3-L4 in the example shown in FIG. 5).

FIGS. 6A and 6B show a different example of user input controls 62 (e.g., interaction handles and/or arrows) for entering desired manipulations to the vertebrae. Circular interaction handles 62 overlaid on the vertebrae can be dragged with mouse or touch screen interaction to alter the angular orientation of the bones across the selected level at the disc space where the arrows intersect. The arrows 62 serve as another manipulation handle; when dragged with mouse or touch screen interaction, the upper vertebra and all vertebrae rostral to it to move linearly in the selected plane relative to the lower vertebra and all vertebrae caudal to it.

In some embodiments, the system can be programmed to run scripted tasks. Some tasks include but are not limited to the following: remove any coronal (Cobb) angles from all levels, making the alignment straight in the coronal plane; for devices at multiple levels, apply a total angular change defined by one input value and distributed evenly throughout instrumented levels. For example, increase lordosis by 6° across 3 implants, resulting in 2° applied to each instrumented level for a net total of 6°.

In some embodiments, a log of the applied changes in angles and displacements is provided to the user as feedback while making adjustments to the positions and orientations of the vertebrae. The log can show changes to individual levels and/or a net (sum) change to all the levels. With this feature, the user knows how much change they have applied at a particular level and can then apply the opposite change at one or more other levels to compensate such that global balance, defined as the net global change in angles and displacements, remains unaffected (zero).

In some embodiments, measurements of segmental angles and disc space heights can be provided to the user for feedback while making adjustments to the positions and orientations of vertebrae. These displayed angles and heights provide absolute values in contrast to the relative values provided by the angle/distance change log just described.

The purpose of adjustment of anatomy is to position the vertebrae where the surgeon user would like them to be post-surgically. In some embodiments, the user would select operative levels (pair of vertebrae and the disc between them) first, then the software would limit the user's ability to manipulate vertebral orientations and positions at the adjacent non-operative levels. In some embodiments, an assumption can be made that the spine will naturally compensate when surgery adjusts angles, and to a lesser extent displacements, of operative levels. That is, a user may assume that, over time, any changes made to the posture of certain levels of the patient's spine will be naturally compensated at other levels with no implants such that once the patient stands up, the net change in posture will be zero due to normal biomechanics. If the user would like to make such an assumption, software may algorithmically apply biomechanical compensation automatically, distributing the negative of any net change in posture at an instrumented level to the levels that are not instrumented. Such a distribution of motion could be even or distributed more to levels that are more caudal. If a physics model of the spine is available, such a model could predict how the spine would compensate for surgery and how much opposite change in angle, if any, should be expected at the non-operative levels. In some embodiments, software may include a setting that is user-selectable, through which the user can request no automatic compensation, compensation distributed evenly to all levels, compensation distributed according to user-defined proportions, or compensation according to a physics model.

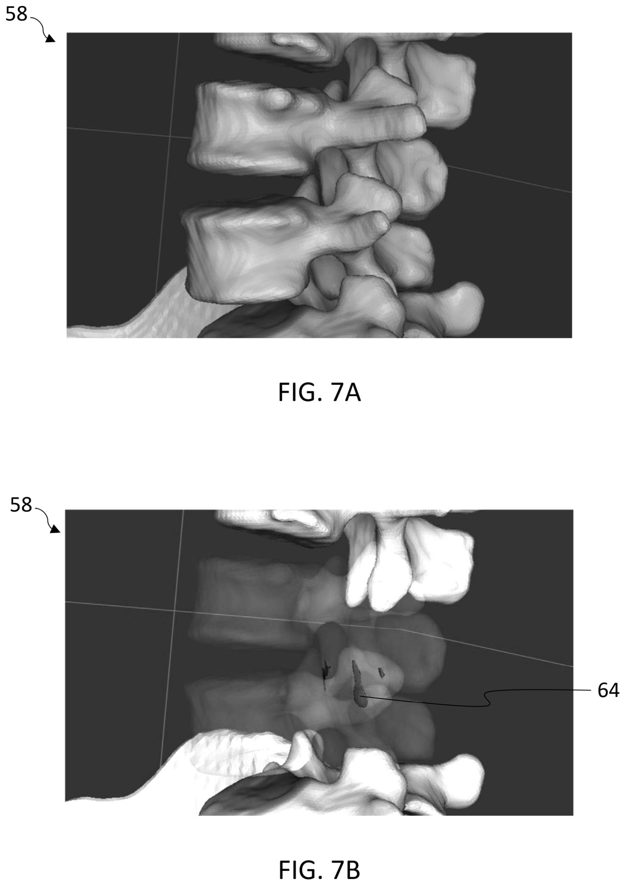

When a surgeon plans to adjust positions and angles of vertebrae and to fuse them in this new orientation, preparatory work may be needed to achieve the desired bony alignment. Bony processes may be in the way of the desired target position of the vertebrae. In some embodiments, software may show collisions between adjacent bones that may occur if the surgeon attempts to move bones into a target position before or while inserting the implant. With further emphasis on FIGS. 7A-7B, the 3D spine image 58 may include one or more collision indicators 64 to identify any potential collisions upon movement of the bones. For example, the vertebrae may be shown as semi-transparent to allow visualization of the collision areas 64 as shown in FIG. 7B. In some embodiments, software can display and draw attention to any bony collisions so that the surgeon knows they will need to prepare the area of the collision during surgery by drilling or excising bone. In some embodiments, software shows the collision of two adjacent vertebrae with indicator(s) 64, such as areas in a red color where intersection is occurring. Simultaneously, to allow easier viewing, the vertebrae involved in the collision can be made transparent while the collision is being monitored. A software feature may allow any regions of bony collision to be recorded as a visual snapshot that can be referenced at the time of surgery. FIG. 7A shows the initial spinal segment without indicating the bony collision(s).

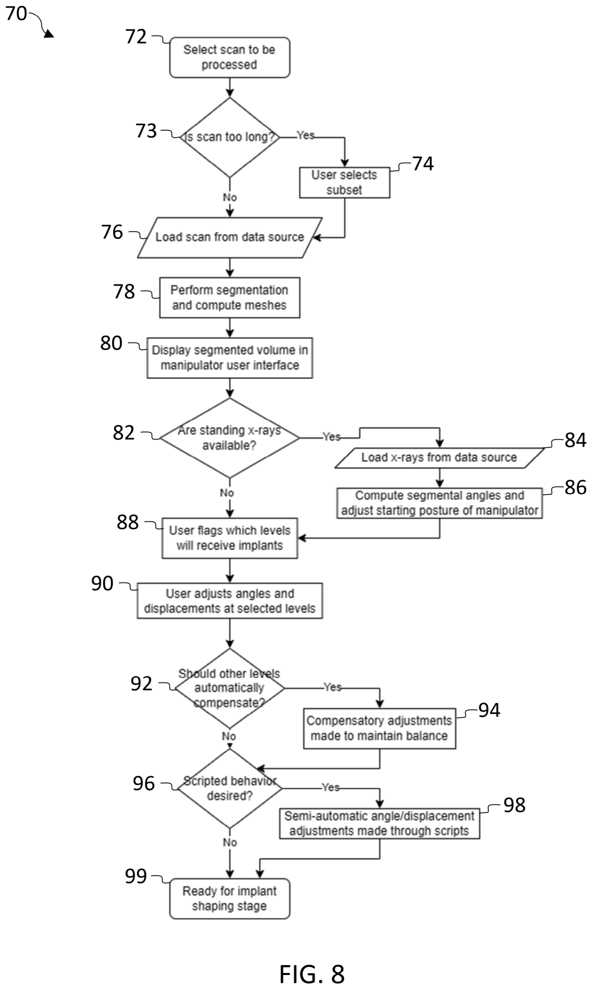

Turning now to FIG. 8, a flowchart 70 of a process for changing spine alignment is shown, according to some embodiments. The scan to be process is selected at step 72 by the user and/or system. If the scan is taking too long (at step 73), the user may select a subset at step 74, and the scan may be loaded from the data source at step 76. If the scan is not taking too long (at step 73), the scan may be loaded from the data source at step 76. At step 78, the segmentation is performed and the meshes are computed for the virtual 3D spine model. At step 80, the segmented volume may be displayed in a manipulator user interface. The user may be asked if standing X-rays are available at step 82. If standing X-rays are available, the X-rays may be loaded from the data source at step 84. At step 86, the segmental angles may be computed and the starting posture of the manipulator may be adjusted based on this information. The user can flag which levels are intended to receive interbody implants at step 88. If the standing X-rays are not available, the user may proceed with the available model to flag which levels are intended to receive implants at step 88. At step 90, the user is able to adjust the angles and displacements at the selected levels. The system and/or user may determine if other levels should automatically compensate for the adjustments at step 92. If other levels should compensate, the compensatory adjustments are made to maintain balance at step 94, and determination of the scripted behavior is made at step 96. If other levels should not compensate, no adjustments are made, and the determination of the scripted behavior is made at step 96. If the scripted behavior is desired, semi-automatic angle/displacement adjustment are made through scripts at step 98, and the implant is ready for the shaping stage at step 99. If the scripted behavior is not desired, no adjustments are made, and the implant is ready for the shaping stage at step 99.

Custom Interbody Implant(s): After the goal and the intervention(s) to achieve the goal has been approved/modified, the system may use the output meshes of the superior and inferior endplates extracted at the segmentation stage to begin forming the contours of the custom interbody. Using a bound of footprint sizes (length x width), the interference between the mesh and footprint forms the final endplate contours of the 3D spacer. By combining the height/angle restoration of the segment (joint) and the extracted endplate contours, a custom virtual implant model may be created in step 18.

In some embodiments, the custom implant can be static, filling the volume of the corrected segment. In some embodiments, the custom implant can be expandable, using a chassis from a matrix of defined footprints and custom expandable endplates (e.g., superior endplate following the contour of the inferior endplate of the cephalad vertebral body, and the inferior endplate following the contour of the superior endplate of the caudal vertebral body.)

After the user has finished inputting changes to the alignment of the spine, the implant shaping stage may take place. Within the portal, the surgeon can select the operative levels of the spine and the angle of approach. The surgeon can then input specific dimensional parameters that they would like the implant to have in order to achieve their desired surgical plan. Those parameters can include, but are not limited to, static or expandable spacer, anterior-posterior depth, medial-lateral width, anterior height, posterior height, lordotic angle, and coronal angle. Additionally, if designing an expandable spacer, the user can select a desired starting height based on any known anatomy or access limitations. Implant starting size including length and width may also be selected automatically. The software may allow each surgeon to maintain a profile containing their preferred parameters and allow them to automatically select them for each case, minimizing the time required to design the implant.

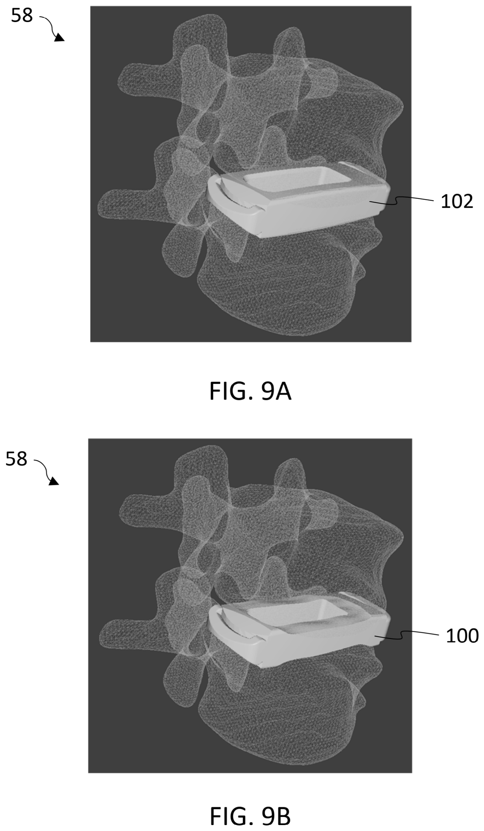

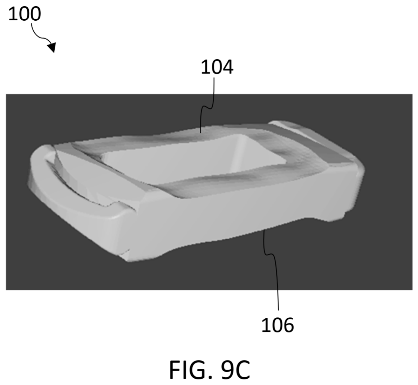

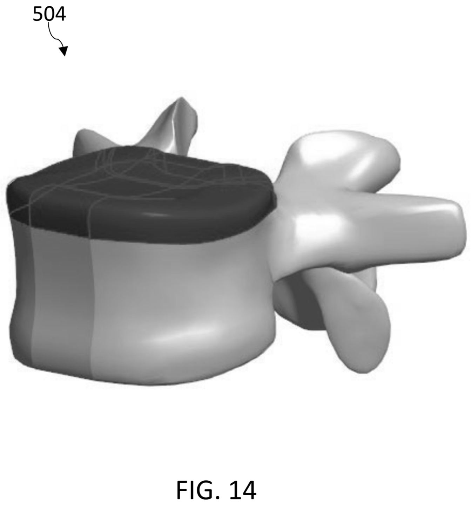

In some embodiments, implant shaping may be done one level at a time to form the interbody implant 100. As shown in FIGS. 9A-9C, the two vertebrae around the level to be shaped may be used as negatives, which are subtracted from a selected implant blank 102 that is slightly too large to fit in the disc space. In other words, the surrounding vertebrae are used to reshape the implant blank 102 into the virtual interbody implant 100. The virtual 3D model 58 of the patient's spine may include a mapping of the topography of the adjacent vertebral endplates. The virtual implant blank 102 may be positioned between the vertebrae such that the implant blank 102 has a height greater than the disc space. The implant blank 102 may then be reshaped by representing it as a mesh and using mesh boolean operations to subtract the intersecting bone meshes from the implant mesh. In other words, the topography of the surrounding vertebrae is used to reshape the custom interbody implant 100. The device mesh may be sized slightly taller than the disc space into which it will be inserted and the surrounding bone meshes are subtracted, creating the interbody implant with custom contoured surfaces contacting the patient's vertebral endplates at the desired height and angle. In another embodiment, the surfaces of the vertebrae may be used as a boundary and the implant is extruded up to the boundary to generate the volume and surface of the interbody implant. In yet another embodiment, each selected vertebrae may be mapped 504, for example, as depicted in FIG. 14. The mapped topography 504 may include a detailed and precise representation of the surface contours (e.g., shapes, curvatures, depressions, or other anatomical variations) of the superior and/or inferior surfaces of the relevant vertebral bodies. The mapped topography 504 of the adjacent vertebrae may be mirrored or otherwise transferred to form the customized upper and lower surfaces 104, 106 of the implant 100 for the patient.

In some embodiments, it may be desirable to reshape the top and bottom surfaces of an expandable interbody such that the interbody produces the desired slope and height once fully expanded but not during the insertion phase. In some embodiments, the top and bottom pieces of the expandable interbody can be provided as separate meshes. Knowing the path of expansion of the mechanism between these parts, the relative locations and orientations of the parts at any point along the expansion path can be predicted as a function of expansion height. The two parts are set to a particular point (expansion height) along the path and both parts are contoured by subtracting the adjacent bone mesh. Alternatively, the parts are set to their starting height and can be automatically adjusted to the position where the desired amount of material contacting the endplates is contoured away. Software can then inform the user what position of expansion of the device needs to be set intraoperatively to achieve the configuration modeled in the planning software.

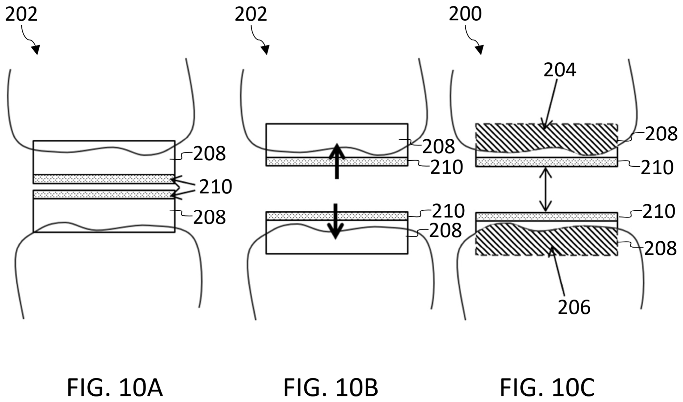

Turning now to FIGS. 10A-10C, in some embodiments for generating an expandable spacer 200, the top and bottom expanding pieces are each represented as two separate meshes 208, 210 (total of four meshes). In this case, the blank 202 may include software meshes having a modifiable portion 208 and an unmodifiable portion 210. The two meshes 210 closer to the center and the expansion mechanism represent the base of the implant and may be unmodifiable 210. Conversely, the two outer meshes 208 represent the portion of the device that can be shaped to adjacent bone as needed, thereby forming the respective upper and lower surfaces 204, 206 of the implant 200. The two upper meshes 208, 210 are placed in apposition and are moved as a unit toward the upper vertebra; similarly, the modifiable and unmodifiable mesh pair 208, 210 for the lower device half are also placed in apposition and move as a unit toward the lower vertebra. The meshes 208, 210 are positioned within the disc space and software can automatically move the mesh pairs 208, 210 vertically upward or downward while monitoring mesh collisions. If the upper mesh pair 208, 210 reaches a position where the unmodifiable mesh 210 is starting to intersect with the upper vertebra, that position is too far and software backs down to a point where the unmodifiable mesh 210 no longer intersects but the modifiable mesh 208 has good coverage across the bony surface. If the lower mesh pair 208, 210 reaches a position where the unmodifiable mesh 210 is starting to intersect with the lower vertebra, that position is too far and software backs up to a point where the lower mesh 208, 210 no longer intersects but the modifiable mesh 208 has good coverage across the bony surface.

More specifically, FIG. 10A shows top and bottom moving pieces that are each represented as a pair of meshes 208, 210, one modifiable mesh 208 where bone will contour and one unmodifiable mesh 210 that cannot be cut for structural integrity. FIG. 10B shows pieces are positioned where the unmodifiable meshes 210 are just on the brink of intersecting with the bone meshes. If while positioned in this way, the modifiable mesh 208 has good contact with bone, then the device design should be suitable. Finally, FIG. 10C shows that when positioned in the target location, the bone meshes are subtracted from the modifiable mesh 208, leaving a contoured piece that can be manufactured via 3D printing or machining. The custom contoured surfaces 204, 206 are configured to exactly match with the adjacent vertebral endplates of the patient. By following this process, the exact height to which the implant 200 should be expanded to achieve the target alignment that the surgeon has planned is known and reproduced on the day of surgery.

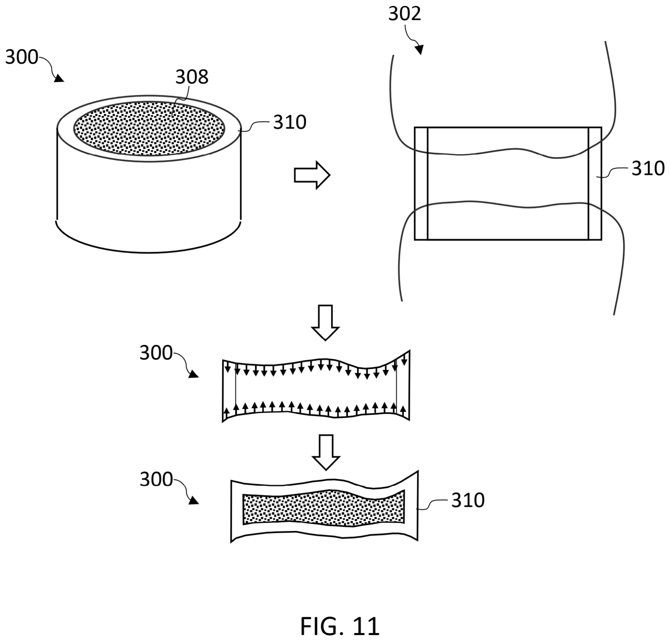

Turning now to FIG. 11, an alternative contoured spacer 300 that has an outer shell portion 310 in contact with bone is also contemplated. In this process, the spacer blank 302 is represented as a mesh comprised of a simple vertically oriented wall shell 310 with or without porous filler 308 and tall enough that bone intersects with the entire cross section. If the bone mesh were simply subtracted from the spacer mesh, it would leave a spacer with no end caps and poor structural integrity horizontally. Instead, the mesh of the contoured device 300 may be converted to a voxelized volume. Then, traveling in rows and columns across the contoured surface, every exposed voxel on the intersection surface of the device is found and vectors are extended toward the device by some thickness, with every voxel intersected given a voxel value representing a solid piece. When the voxelized volume is then converted back into a mesh, it will have a shell 310 covering and reinforcing the ends of the device 300. The resulting object may have a porous center 308 with a solid outer shell 310.

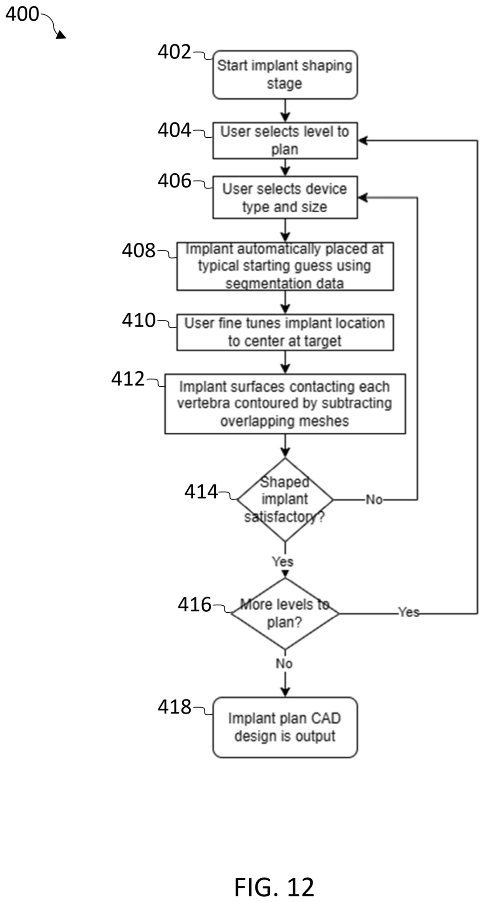

FIG. 12 shows a flowchart 400 representative of the implant shaping stage, according to some embodiments. Once the spinal alignment corrections are complete, the spine may be re-segmented and the CAD model of the virtual spine is exported. The model is then imported into a CAD program (e.g., CREO, Solidworks, etc.) and a user selects the bony surfaces above and below an operative level. Those surfaces are then used to shape the interbody implant by cutting away material or extruding material to the surfaces as described herein. The final implant model is then placed into the virtual spine and imported back into the software and presented to the user for their approval. The flowchart 400 may include starting the implant shaping stage at step 402, and the user selects the level to plan at step 404. The user selects the device type and size at step 406. At step 408, the implant is automatically placed at a typical starting setting using segmentation data. At step 410, the user fine tunes the implant location to center at a target. The implant surfaces contacting each adjacent vertebra are contoured by subtracting overlapping meshes at step 412. Then, at step 414, the user determines if the implant is shaped properly. If not, the user returns to device size and type at step 406 in the process to try a different implementation and prior steps are repeated. If properly shaped, then the user determines if there are additional levels to plan at step 416. If yes, the process returns to selecting the additional level to plan (step 404). If no, the implant plan design output (e.g., CAD design) is generated at step 418.

After the custom implant generation, CAD model handling may include providing the patient's data including the implant models to a robotic navigation system, such as ExcelsiusGPS, to utilize and/or update the data throughout the intra-operative procedure.

Case Data Creation: First, a patient's data may be saved within the surgeon's dashboard/profile. Within the patient's profile, the computer saves the patient's images, correction goals, custom implant CAD models, and the positions and trajectories of those implants. The digital case data or pre-operative plan can be used with the surgical navigation system.

CAD Model Export: The computer program exports the patient images, CAD models of custom interbodies, shapes of initial and target spine, and the positions of interbodies and trajectories to digital case data that can be used with a surgical navigation device.

Case Import: The user loads the case data in computer software on the surgical navigation device to start the surgical steps.

Intra-operative Registration: The user registers intra-operative patient anatomy to the pre-operative 3D scans using techniques such as intra-operative Fluoroscopy or intra-op CT. The user re-registers intra-operative images to the pre-operative 3D scans during the surgery as needed, since the spine shape may differ from pre-operative images due to patient positioning.

Progress Evaluation: Computer software modifies the shape of spine according to registration of 3D images with intra-operative images. Computer software also re-computes spine measurements. Computer software displays the shape and measurements with an opportunity for the user to compare it to initial and target shapes. Using surgical navigation, the positions of vertebral bodies are tracked using techniques such as optical markers attached to each vertebra or vertebra surface tracking. As the implant expands, the positions of vertebral bodies change. The computer software displays the placement and expansion of the implant based on the initial and final position of the vertebral bodies, CAD model of implant, and mechanical expansion mechanism of the implant.

Post-Operative Data Collection: All the data collected within the procedure including progress images, various tool paths including implant inserters, and final images are saved with the patient profile for future retrieval and outcomes studies.

In addition to interbody implants, implant fixation (e.g., screws) can further bind the surgical site to augment stability where instability has been added (e.g., due to bony removal, disc removal, and/or tissue resection) to achieve the correction goal. Screws may be auto-filled into the pedicle based on the anatomical constraints (e.g., minimum pedicle cross-section, pedicle shape, vertebral body depth, etc.) and further refined by the surgeon's surgical approach preference (e.g., pedicle for open approach, paramedian for MIS approach, midline for cortical). Screws may then be optimized to minimize the required energy to bend a rod and for ease of rod introduction (e.g., the rod contour may be minimized by adjusting screw locations medial-lateral to be in line with the adjacent segments).

Rod: In some embodiments, a rod may be fit through the spline of the screws where the screws represent the final positions. The rod may be designed such that it is laid into the screwheads without requiring any further reduction and/or maneuvering. In some embodiments, the rod may be designed such that it will drive additional correction and be contoured with an over bend to adjust for reduction forces which result in "rod flattening."

Surgical Intervention: The custom implants may be implanted using navigation if registered within a robotic and navigation system, such as the ExcelsiusGPS ecosystem. In some embodiments, the custom implants may be implanted freehand according to surgeon preference.

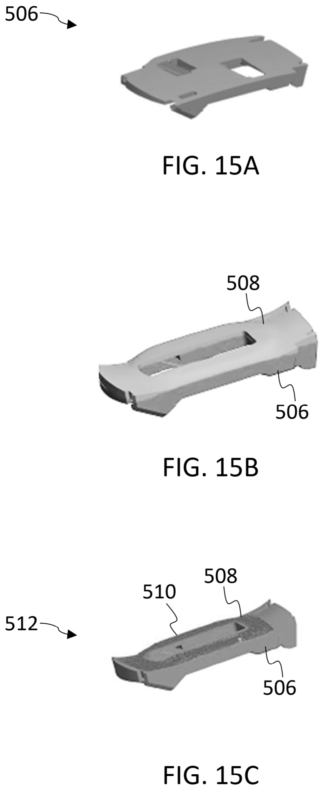

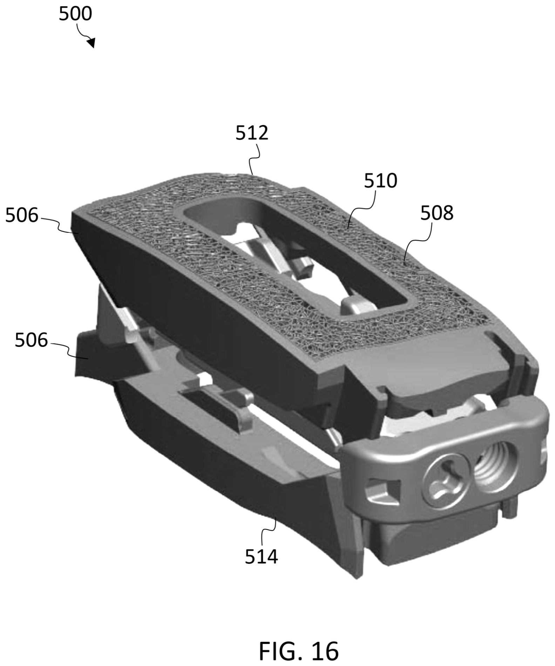

In some embodiments, the custom patient-specific interbody implants are manufactured in step 22 (of FIG. 2). FIGS. 15A-15C depict one example of manufacturing custom patient-specific expandable implant 500. In this embodiment, after the topography of the endplates of the vertebral body are mapped 504 (e.g., as shown in FIG. 14), then the patient-matched surface 508 is applied to the interbody implant 500. In particular, one or both of the expandable interbody spacer implant endplates 512, 514 receive the patient-matched surface 508. As best seen in FIG. 15A, the implant endplates 512, 514 may start as a blank 506 with a base or support structure including the ramped surfaces. The patient-matched surface 508 may be applied to the vertebral-facing outer surface of the blank 506, for example, via 3D printing. Once the patient-matched surface 508 is applied, the endplate 512 may optionally be filled in with a lattice or porous structure 510 (e.g., a trabecular bone-like lattice), for example, as depicted in FIG. 15C, to create a fully porous endplate 512, 514 that allows for bony on-growth and ingrowth. FIG. 16 depicts the fully assembled expandable implant 500 including top and bottom endplates 512, 514 with the custom patient-matched surfaces 508 and the lattice structure 510. Endplates that match the anatomy of the spine can better distribute load to help heal the spine, reduce complications, and improve surgical outcomes. Expandable spacer technology helps restore many of the surgical parameters surgeons are interested in while preserving the integrity of the vertebral body endplate with minimized impaction.

The implant components may be manufactured in any suitable way. For example, the endplates 512, 514 may be completely 3D printed and then machined to include the ramp features that facilitate expansion. Alternatively, each part may be fully 3D printed and then assembled. In another instance, the blank 506 may be machined to include the ramped surfaces. The machined endplate blank 506 may be fixtured in a 3D printer, and the added lattice 510 and topography 508 may be subsequently printed on the blank 506 creating the complete patient-matched endplate 512. This process may also be used for the other implant endplate 514, and then both endplates 512, 514 can be assembled to the expansion mechanism, thereby forming the expandable implant 500. It will be appreciated that any suitable manufacturing method may be selected to form any suitable custom patient-specific implant type.

Patient-Specific Integrated ALIF Spacers: Also provided herein are methods of inserting/implanting patient-specific integrated ALIF spacers using an in-line impaction of anchors with the patient-specific implant.

Placing screws into an ALIF spacer can be difficult due to the angle of trajectory often being blocked by the pubis symphysis, especially if the integrated spacer is at the L5/S1 level and when trying to place the superior screw. Additionally, with all the vessels present, having to enter the incision space multiple times for each screw hole to awl, drill, tap, and place the screw using sharp instrumentation can increase the risk of vessel damage. Cage/implant insertion and screw preparation can be even more challenging when other spinal deformities are present. Allowing for patient-specific implants to be compatible with anchor fixation and in-line anchor barrel guide inserters, as described below, can streamline and improve the safety of inserting the cage and fixation by allowing the anchors to be contained in the inserter such that no sharp instruments need to be passed through the incision site. Additionally, the in-line anchor barrel guide enables the fixation to be deployed despite any challenging anatomy, including interference with a high pubis symphysis or challenge reaching the fixation hole angulation for screws, as the anchors are deployed through and in-line with the inserter.

Accordingly, insertion of a patient-specific implant and anchors may be executed using an in-line anchor barrel guide that acts as an inserter for the implant into the disc space. The in-line anchor barrel guide would also house the anchors that are then impacted into the implant and vertebral body. The anterior face of the implant comprises an attachment mechanism that allows it to removably couple to the distal end of the inserter. Once the implant is attached, the anchors may be placed in the in-line anchor barrel guide inserter to be deployed once the cage/implant is inserted.

Once inserted, anchor impactors can travel down the length of the inserted to push the anchors being housed in the in-line anchor barrel guide inserter into the cage and vertebral body. After the anchors are deployed, the inserter can be removed from the implant.

Scaling Calibration Methods: Also provided herein are methods for scaling patient images. Many imaging modalities (2D and 3D) help view, understand, and diagnose a patient's anatomy. In the case of musculoskeletal pathologies, 2D radiographs are often the first image modality to be ordered by the clinician. If more comprehensive evaluation of bony anatomy is desired, 3D CT scans may be further ordered. In orthopedics, clinicians may rely on various measurements of the patient's bony anatomy to correctly understand the patient's alignment and posture determine treatment and plan surgery. To carry out these measurement on 2D radiographs clinicians increasingly rely on digital tools to interactively draw lines and angles. Certain measurement describe absolute distances between structures, and therefore require knowledge of the scaling (i.e., the real-world distance represented between two adjacent pixels) in the radiograph. Oftentimes, this information can be obtained through radiopaque calibration markers present in the radiograph at the time of image capture. These marker are of known size, and the clinician simply needs to draw a line matching the dimension of the marker to relate the scaling to a real-world distance. However, in some cases, these scaling calibration markers may be absent from the image.

In some embodiments, methods to automatically calibrate 2D radiographs for a patient who possesses calibrated 2D and/or 3D CT imaging are provided. This may help clinicians make accurate radiographic measurements when they have an uncalibrated 2D radiograph with no known distances in the image for patients with non-osteoporotic bone. The scaling calibration methods may relate known scaling information in a 2D or 3D image, to an image of the same patient with unknown scaling information. The approach can help clinicians compute distance-based measurements in 2D radiographs without meaningful scaling information.

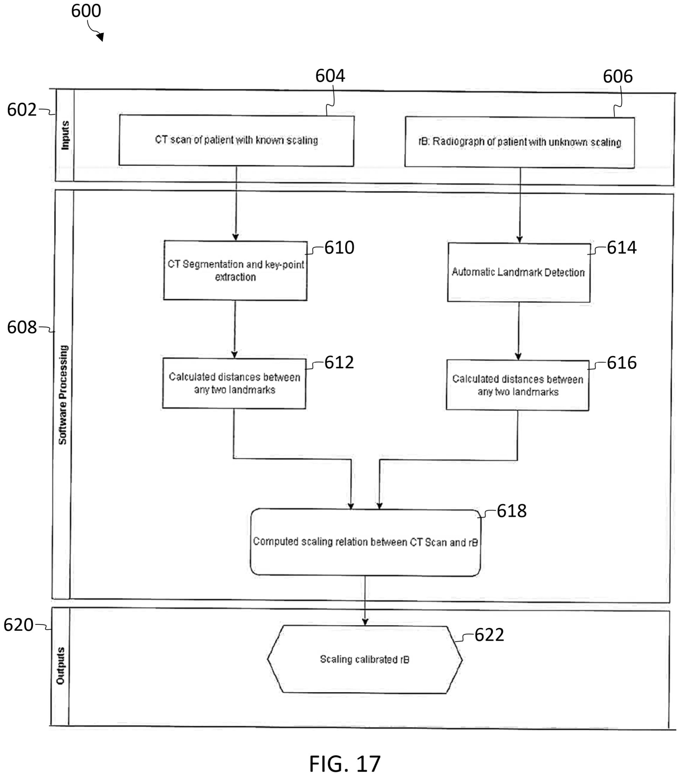

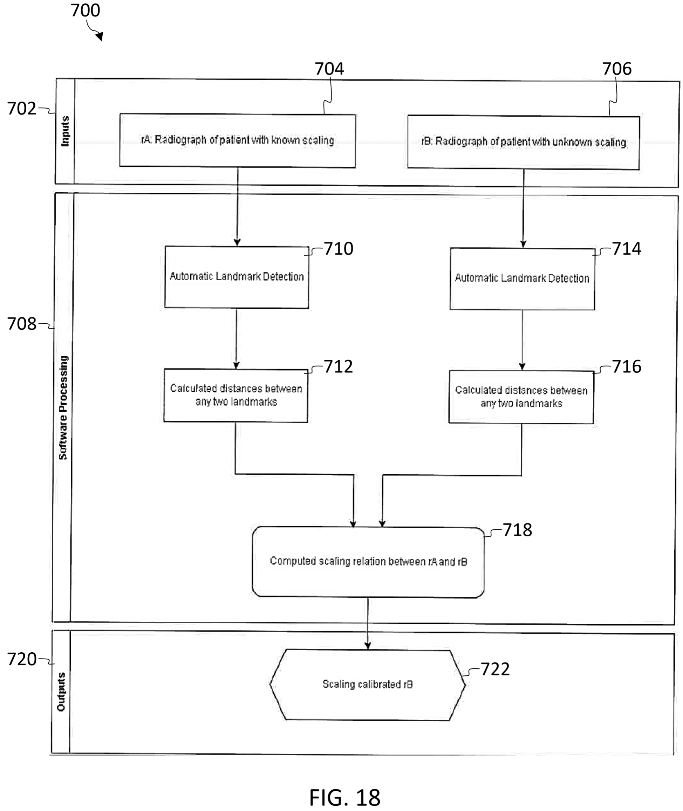

FIGS. 17 and 18 depict scaling calibration methods 600, 700 for radiographs with unknown scaling. In FIG. 17, the scaling method 600 converts a CT scan with a known scaling 604 and a radiograph with an unknown scaling 606 into a calibrated radiograph 622. The scaling method 600 starts with inputs 602 including the CT scan of a patient with known scaling 604 and the radiograph of a patient with unknown scaling 606. The software processing 608 includes CT segmentation and key-point extraction 610 of the CT scan, and calculated distances between any two landmarks 612 on the CT scan. For the radiograph with unknown scaling 606, the software processing 608 includes automatic landmark detection 614 and calculated distances between any two landmarks 616. Then, the software processing 608 includes a computed scaling relation between the CT scan and radiograph 618 based on the calculated distances between landmarks 612, 616 present on both images 604, 606. The output 620 provides the scaling calibrated radiograph 622. In FIG. 18, the scaling method 700 converts a radiograph with a known scaling 704 and a radiograph with an unknown scaling 706 into a calibrated radiograph 722. The scaling method 700 starts with inputs 702 including the radiograph of a patient with known scaling 704 and the radiograph of a patient with unknown scaling 706. The software processing 708 includes automatic landmark detection 710 of the radiograph, and calculated distances between any two landmarks 712 on the radiograph. For the radiograph with unknown scaling 706, the software processing 708 includes automatic landmark detection 714 and calculated distances between any two landmarks 716. Then, the software processing 708 includes a computed scaling relation between the radiographs 718 based on the calculated distances between landmarks 712, 716 present on both images 704, 706. The output 720 provides the scaling calibrated radiograph 722.

The 2D image calibration method allows clinicians to make distance-based measurements on 2D radiographs that don't have useful distance information if a calibrated 2D radiograph or a 3D CT image of the same patient is available. In the case of spinal surgery, the clinician might be interested in measuring certain alignment parameters like the Sagittal Vertical Axis (SVA) or Leg Length Discrepancy (LLD). Using this method, the scaling calibration can be done automatically if another calibrated 2D or 3D image is available. This method is additionally useful in the case of unscaled EOS imaging. The EOS imaging system captures low-dose full body AP and Lateral radiographs simultaneously. This simultaneous capture allows for a 3D reconstruction of the patient spine, enabling better visualization of 3D curvatures, improved preoperative planning and enhanced patient education. The scaling calibration method allows for a 3D reconstruction using uncalibrated EOS imaging if other 2D or 3D patient imaging is available.

Any references cited herein are expressly incorporated by reference in their entirety. There are many different features to the present disclosure and it is contemplated that these features may be used together or separately. Unless mention was made above to the contrary, it should be noted that all of the accompanying drawings are not to scale. Thus, the disclosure should not be limited to any particular combination of features or to a particular application of the disclosure. Further, it should be understood that variations and modifications within the spirit and scope of the disclosure might occur to those skilled in the art to which the disclosure pertains. Accordingly, all expedient modifications readily attainable by one versed in the art from the disclosure set forth herein that are within the scope and spirit of the present disclosure are to be included as further embodiments of the present disclosure.

The invention could be inter alia defined by the following examples:
1. A method of manufacturing a custom patient-specific interbody implant for a patient, the method comprising: uploading images of a spine for a patient into a processor configured to execute software instructions stored on a memory; processing the images into a virtual three-dimensional model of the spine that comprises topography of individual vertebral bodies displaying the virtual three-dimensional model of the spine on an interface connected to the processor; manipulating the virtual three-dimensional model on the interface to pre-operatively simulate a desired correction to the spine; generating a virtual implant model based on the desired correction to the spine and the topography of adjacent vertebral bodies; and manufacturing a custom interbody implant for the patient based on the virtual implant model.
2. The method of Example 1, wherein the images comprise lying CT scans and standing X-rays of the patient.
3. The method of Example 2, wherein it is provided for automatically modifying the virtual three-dimensional model of the spine to represent a standing posture instead of a lying posture of the patient based on the images.
4. The method of Example 1, wherein manipulating the virtual three-dimensional model on the interface comprises indicating on the virtual three-dimensional model of the spine any potential collisions between adjacent bones.
5. The method of Example 1, wherein the virtual three-dimensional model comprises a series of meshes in three mutually orthogonal multi-planar reconstruction views, and the virtual implant model includes a mesh reshaped by the virtual three-dimensional model of the spine.
6. The method of Example 5, wherein the virtual implant model is generated by overlapping a portion of the mesh of the virtual implant model with a portion of the series of meshes of the virtual three-dimensional model such that any intersecting areas are subtracted from the mesh of the virtual implant model to mimic the topography of adjacent vertebral bodies.
7. The method of Example 1, comprising installing the custom interbody implant into the patient based on the virtual implant model and the desired correction.
8. A custom patient-specific interbody implant comprising: a body having an upper surface having an irregular surface topography and a lower surface having an irregular surface topography configured to contact adjacent vertebral bodies, wherein the upper surface and the lower surface which are custom contoured to fit between adjacent vertebral endplates of a specific patient, wherein the irregular surface topography of the upper surfaced and the irregular surface topography of the lower surface are predetermined from a virtual implant model generated by overlapping a portion of the virtual implant model with a portion of a virtual three-dimensional model of a spine of the patient simulated pre-operatively with a desired correction to the spine, such that any intersecting areas are subtracted from the virtual implant model to mimic the topography of the adjacent vertebral endplates of the patient, and wherein the virtual three-dimensional model of the spine of the patient is simulated based on segmentation of pre-operative two-dimensional and three-dimensional imaging of the patient, thereby converting the virtual three-dimensional model of the spine to represent a standing posture instead of a lying posture of the patient.
9. The implant of Example 8, wherein the upper surface and the lower surface are constructed from a mapped topography of the adjacent vertebral endplates for the specific patient and filled in with a trabecular lattice.
10. The implant of Example 8, wherein the implant is a static implant
11. The implant of Example 8, wherein the implant is an expandable implant.
12. The implant of Example 10, wherein the expandable implant includes an upper endplate defining the customized upper surface, a lower endplate defining the customized lower surface, and a central actuator with ramps configured to expand the upper endplate and the lower endplate.
13. The implant of Example 12, wherein the upper endplate and the lower endplate are each represented in the virtual implant model as two separate meshes each comprising a modifiable mesh portion that is subtracted to form the irregular surface topography and an unmodifiable mesh portion that is retained for structural integrity of the upper and lower endplates.
14. The implant of Example 8, wherein the implant comprises a solid shell surrounding an inner porous center.
15. A method of generating a custom implant comprising: uploading two-dimensional and three-dimensional images of a portion of a patient's spine; processing the images on a processor configured to execute software instructions stored on memory; registering the images; creating a patient-specific kinematic model that estimates joint forces of each motion segment of the patient's spine; automatically suggesting spinal modifications; manually modifying the automatically suggested spinal modifications and/or accepting the automatically suggested spinal modifications; generating a custom implant based on the automatically suggested spinal modifications including any manual modifications.
16. The method of Example 13, wherein processing the images comprises detecting one or more of patient anatomy or regions of interest and automatically measuring global spine parameters and local spine parameters.
17. The method of Example 15 wherein registering the images comprises registering two anatomical plane images to one origin to establish an initial three-dimensional coordinate system and then segmented levels are registered to the initial three-dimensional coordinate system.
18. The method of Example 15, wherein automatically suggesting spinal modifications includes software instructions which provide a corrected spine model based on a machine-learning model.
19. The method of Example 15 wherein the patient-specific kinematic model is determined from the images and inputs concerning the patient including demographic data, anthropometric data, and physiological and biological data.
20. The method of Example 15 wherein preferably a first three-dimensional image of the portion of the patient's spine is registered to a first two-dimensional image of the portion of the patient's spine.

## Claims

1. A method of manufacturing a custom patient-specific interbody implant for a patient, the method comprising:
- uploading images of a spine for a patient into a processor configured to execute software instructions stored on a memory;
- processing the images into a virtual three-dimensional model of the spine that comprises topography of individual vertebral bodies;
- displaying the virtual three-dimensional model of the spine on an interface connected to the processor;
- manipulating the virtual three-dimensional model on the interface to pre-operatively simulate a desired correction to the spine;
- generating a virtual implant model based on the desired correction to the spine and the topography of adjacent vertebral bodies; and
- manufacturing a custom interbody implant for the patient based on the virtual implant model.

2. The method of claim 1, wherein the images comprise lying CT scans and standing X-rays of the patient.

3. The method of claim 2, wherein it is provided for automatically modifying the virtual three-dimensional model of the spine to represent a standing posture instead of a lying posture of the patient based on the images.

4. The method of any one of the preceding claims, wherein manipulating the virtual three-dimensional model on the interface comprises indicating on the virtual three-dimensional model of the spine any potential collisions between adjacent bones.

5. The method of any one of the preceding claims, wherein the virtual three-dimensional model comprises a series of meshes in three mutually orthogonal multi-planar reconstruction views, and the virtual implant model includes a mesh reshaped by the virtual three-dimensional model of the spine.

6. The method of claim 5, wherein the virtual implant model is generated by overlapping a portion of the mesh of the virtual implant model with a portion of the series of meshes of the virtual three-dimensional model such that any intersecting areas are subtracted from the mesh of the virtual implant model to mimic the topography of adjacent vertebral bodies.

7. The method of any one of the preceding claims, comprising installing the custom interbody implant into the patient based on the virtual implant model and the desired correction.

8. A custom patient-specific interbody implant comprising:
- a body having an upper surface having an irregular surface topography and a lower surface having an irregular surface topography configured to contact adjacent vertebral bodies, wherein the upper surface and the lower surface which are custom contoured to fit between adjacent vertebral endplates of a specific patient,
- wherein the irregular surface topography of the upper surfaced and the irregular surface topography of the lower surface are predetermined from a virtual implant model generated by overlapping a portion of the virtual implant model with a portion of a virtual three-dimensional model of a spine of the patient simulated pre-operatively with a desired correction to the spine, such that any intersecting areas are subtracted from the virtual implant model to mimic the topography of the adjacent vertebral endplates of the patient, and wherein the virtual three-dimensional model of the spine of the patient is simulated based on segmentation of pre-operative two-dimensional and three-dimensional imaging of the patient, thereby converting the virtual three-dimensional model of the spine to represent a standing posture instead of a lying posture of the patient.

9. The implant of claim 8, wherein the upper surface and the lower surface are constructed from a mapped topography of the adjacent vertebral endplates for the specific patient and filled in with a trabecular lattice.

10. The implant of claim 8, wherein the implant is a static implant or is an expandable implant.

11. The implant of claim 10, wherein the expandable implant includes an upper
endplate defining the customized upper surface, a lower endplate defining the customized lower surface, and a central actuator with ramps configured to expand the upper endplate and the lower endplate, and wherein preferably the
upper endplate and the lower endplate are each represented in the virtual implant model as two separate meshes each comprising a modifiable mesh portion that is subtracted to form the irregular surface topography and an unmodifiable mesh portion that is retained for structural integrity of the upper and lower endplates.

12. The implant of any one of the claims 8-11, wherein the implant comprises a solid shell surrounding an inner porous center.

13. A method of generating a custom implant comprising:
- uploading two-dimensional and three-dimensional images of a portion of a patient's spine;
- processing the images on a processor configured to execute software instructions stored on memory;
- registering the images;
- creating a patient-specific kinematic model that estimates joint forces of each motion segment of the patient's spine;
- automatically suggesting spinal modifications;
- manually modifying the automatically suggested spinal modifications and/or accepting the automatically suggested spinal modifications;
- generating a custom implant based on the automatically suggested spinal modifications including any manual modifications.

14. The method of claim 13, wherein processing the images comprises detecting one or more of patient anatomy or regions of interest and automatically measuring global spine parameters and local spine parameters and wherein preferably registering the images comprises registering two anatomical plane images to one origin to establish an initial three-dimensional coordinate system and then segmented levels are registered to the initial three-dimensional coordinate system.

15. The method of claim 13, wherein automatically suggesting spinal modifications includes software instructions which provide a corrected spine model based on a machine-learning model and wherein preferably the patient-specific kinematic model is determined from the images and inputs concerning the patient including demographic data, anthropometric data, and physiological and biological data and wherein preferably a first three-dimensional image of the portion of the patient's spine is registered to a first two-dimensional image of the portion of the patient's spine.
